# EUROPÄISCHE PATENTSCHRIFT

(11) **EP 1 977 233 B1**
(45) Veröffentlichungstag und Bekanntmachung des Hinweises auf die Patenterteilung: **07.03.2012**
(21) Anmeldenummer: 07712071.5
(22) Anmeldetag: 22.01.2007
(51) Int. Cl.: G01N 33/00, G01N 27/403

(54) **VERFAHREN UND VORRICHTUNG ZUM MESSEN DER KONZENTRATION EINES GASBESTANDTEILS IN EINEM GASGEMISCH**
METHOD AND APPARATUS FOR MEASURING THE CONCENTRATION OF A GAS CONSTITUENT IN A GAS MIXTURE
PROCEDE ET DISPOSITIF DE MESURE DE LA CONCENTRATION D'UN COMPOSANT GAZEUX DANS UN MELANGE DE GAZ

(30) Priorität: 23.01.2006 DE 102006003560
(43) Veröffentlichungstag der Anmeldung: 08.10.2008
(73) Patentinhaber: Union Instruments GmbH, 76185 Karlsruhe (DE)
(72) Erfinder: HAUG, Torsten, 76889 Dörrenbach (DE); KIENKE, Peter, 77815 Bühl (DE)
(74) Vertreter: Solf, Alexander
(86) Internationale Anmeldenummer: PCT/EP2007/050614
(87) Internationale Veröffentlichungsnummer: WO 2007/082955

(56) Entgegenhaltungen:
- EP-A1- 0 840 117
- WO-A-92/19965
- DE-A1- 4 407 345
- DE-A1- 10 318 956
- DE-A1- 19 808 816
- DE-A1- 19 810 973
- DE-U1- 9 415 809
- JP-A- 2006 284 400
- US-A1- 2003 000 285

## Beschreibung

Die Erfindung betrifft ein Verfahren und eine Vorrichtung zum Messen der Konzentration eines Gasbestandteils in einem Gasgemisch, insbesondere des Schwefelwasserstoffs (H₂S) in einem überwiegend Methan (CH₄) enthaltenden Gasgemisch z. B. in einem Biogas.

Biogas enthält als Hauptbestandteil Methan (CH₄) und wird vermehrt als Energieträger verwendet. Störend wirkt dabei der H₂S-Bestandteil, denn H₂S kann bereits ab 0,035 Vol.-% im Gasgemisch toxisch wirken und korrodiert Metalle stark, so dass H₂S z. B. in Verbrennungsanlagen erhebliche Korrosionsprobleme verursachen kann. Außerdem können auch Messgeräte, Halbleiterelemente oder Gasförderpumpen durch H₂S-Angriff beeinträchtigt werden.

H₂S kann in wechselnden Mengen im Biogas vorkommen. Meist sind 100 - 200 ppm H₂S vorhanden; möglich sind aber auch 5.000 - 10.000 ppm H₂S, wobei die verbrauchende Industrie Produkte wünscht, die weniger als 50 ppm H₂S aufweisen.

Gemessen wird die Konzentration von H₂S in einem Gasgemisch meist mit elektrochemischen Sensoren (ECS). Diese Sensoren haben grundsätzlich einen eingeschränkten sogenannten nominalen Messbereich mit einer Messbereichsgrenze und messen in der Regel nur Konzentrationen bis zu 0,5 Vol.-% mit ausreichender Sicherheit. Den nominalen Messbereich überschreitende Konzentrationen verursachen z. B. eine Sättigung der sensitiven Stoffe des Sensors, so dass die genaue Konzentration nicht ermittelbar ist. Diesen Mangel versucht man mit Verdünnungen des Gasgemisches, z. B. mit Luft, auf erfassbare unterhalb der nominalen Messbereichsgrenze liegende Konzentrationen zu kompensie-ren. Dabei wird z. B. durch Messen der Konzentration des Hauptbestandteils, z. B. von CH₄, sowohl, im Gasgemisch als auch in der Verdünnung sowie des H₂S-Gehalts in der Verdünnung der H₂S-Gehalt im Gasgemisch errechnet. Dieses bekannte Verfahren erfordert komplizierte Ventilanordnungen und den Einsatz sehr genau arbeitender teurer Sensoren (DE 101 56 856 A1). Gleiches aufwendige, auf einer Verdünnung basierende Messverfahren werden in der DE 44 07 345 A1 und der EPO 840 117 A1 beschrieben.

Die sensitiven Stoffe einiger elektrochemischer Sensoren verlieren während der Reaktion mit Gasgemischen Bestandteile, die regeneriert werden können. Sofern im Gasgemisch Stoffe für die Regeneration zur Verfügung stehen, regeneriert sich der sensitive Stoff automatisch und ohne weiteres. Häufig sind diese regenerierenden Stoffe im zu detektierenden Gasgemisch, nicht vorhanden, so dass die Standzeit des Sensors mangels Regeneration verkürzt wird

Einige Sensoren brauchen bestimmte Bestandteile aus dem zu messenden Gasgemisch für Messreaktionen, die in manchen Gasgemischen fehlen. Auch dieser Mangel verkürzt die mögliche Messdauer des Sensors.

Aufgabe der. Erfindung ist, ein Verfahren und eine Vorrichtung zum Messen der Konzentration eines Gasbestandteils in einem Gasgemisch, insbesondere H₂S in vorzugsweise einem Biogas, mit mindestens einem elektrochemischen Sensor zu schaffen, die bei möglicher Überlastung des Sensors z. B. bei möglichen Überschreitungen des nominalen Messbereichs genaue Konzentrationsmessungen ohne Verdünnungsverfahren ermöglichen.

Diese Aufgabe wird durch die Merkmale der Ansprüche 1 und 17 gelöst. Vorteilhafte Weiterbildungen der Erfindung werden in den von diesen Ansprüchen abhängigen Unteransprüchen gekennzeichnet.

Die Erfindung ermöglicht vorzugsweise die Messung eines Gasbestandteils bis 10000 ppm, insbesondere bis 1000 ppm H₂S und macht sich die Eigenart elektrochemischer Sensoren zunutze, wonach die Sensoren aufgrund chemischer Reaktionen an einer Messelektrode eine im wesentlichen konzentrationsabhängige, relativ träge Ansprechzeit und eine relativ träge Abklingzeit der Reaktionsmessung haben und dass die Betriebsdauer abhängig ist von der Belastung des Sensors. Ein Sensor, der beispielsweise im Bereich der Messbereichsgrenze seines nominalen Messbereichs betrieben wird, ist z. B. nach wenigen Wochen verbraucht und muss ausgetauscht werden. Wird ein Sensor dagegen z. B. in einem Sensormessbereich zwischen 10 und 20 % seines nominalen Messbereichs betrieben, kann seine einwandfreie Messleistung auf mehrere z. B. zwei Jahre verlängert werden.

Nach der Erfindung wird der Sensor mit einem bestimmten unverdünnten strömenden Gasgemischvolumen einer Gasart und strömenden. Trägergasvolumen einer anderen Gasart im Wechsel beaufschlagt, wobei in einer Gasleitung des Messgerätes ein vorbestimmtes, relativ kleines Gasgemischvolumen mit Phasengrenzen zwischen zwei größeren Trägergasvolumina stoßweise bzw. gepulst in das hilfsweise verwendete strömende Trägergas eingefügt wird und mit einer Aufeinanderfolge der strömenden Gasvolumina der verschiedenen Gassorten (Trägergas, z. B. Luft, und Gasgemisch, z. B. Biogas, einschließlich zu detektierendem Gasgemischbestandteil, z. B. H₂S) der Sensor beaufschlagt wird, und zwar in der Regel in einer Gasgemischvolumenmenge, die eine Art von der Gasleitungswandung und dem Trägergas begrenztes Gasgemischpolster mit Phasengrenzen zum Trägergas bildet, und während einer bestimmten Anströmdauer, die noch keine vollständige der tatsächlichen Konzentration entsprechenden Reaktion des sensitiven Stoffes im Sensor bewirkt und somit ein Messwert erzeugt wird, der in einem bestimmten Messbereich unterhalb der nominalen Messbereichsgrenze, z. B. in einem Messbereich von 10 - 20 % des nominalen Messbereichs bezüglich des zu messenden Gasbestandteils des Gasgemisches liegt.

Auf diese Weise wird zwar kein der tatsächlichen Konzentration des Gasbestandteils im Gasgemisch entsprechender Messwert gemessen; es wird vielmehr mit einer von der Anströmvolumezzmenge und der Anströmzeit des Gasgemisches abhängige, kleinere Messgröße gemessen, die z. B. in Form einer kleineren Amplitude und/oder einer kleineren Messkurvenfläche angezeigt wird. Aufgrund einer vorherigen Eichung des Messgerätesensors wird erfindungsgemäß auf die genaue Konzentration des Gasbestandteils im Gasgemisch rückgerechnet. Dies erfolgt mit einer entsprechenden Software, die ein dafür verwendetes elektronisches Steuergerät verarbeitet.

Nach einer besonderen Ausführungsform der Erfindung erfolgt die Messung, indem zeitlich in bestimmten kurzen Abständen getaktet bzw. gepulst mehrmals Gasgemischvolumina in das Trägergas eingebracht werden, und zwar mit einer Frequenz, die ein merkliches bzw. beachtliches Abklingen der Reaktion der sensitiven Stoffe des Sensors verhindert, so dass der Messwert des Messgerätes relativ konstant auf einem bestimmten Niveau erhalten bleibt. Dadurch wird die Genauigkeit der Bestimmung der Gasbestandteilkonzentration erheblich erhöht.

Als Trägergas wird ein Gas verwendet, das den zu detektierenden Gasgemischbestandteil nicht enthält bzw. in nicht detektierbaren geringen Mengen enthält und insofern inert ist.

Das erfindungsgemäße Verfahren und die erfindungsgemäße Vorrichtung werden im folgenden anhand der Zeichnungen beispielhaft näher erläutert. Es zeigen:
- Fig. 1a-d: schematisch und idealisiert das erfindungsgemäße Messprinzip;
- Fig. 2: schematisch Einrichtungen einer erfindungsgemäßen Messvorrichtung;
- Fig. 3: schematisch einen Längsschnitt durch eine erfindungsgemäße Messkammer.
- Fig. 4: schematisch den Aufbau eines erfindungsgemäß verwendeten Ventils.

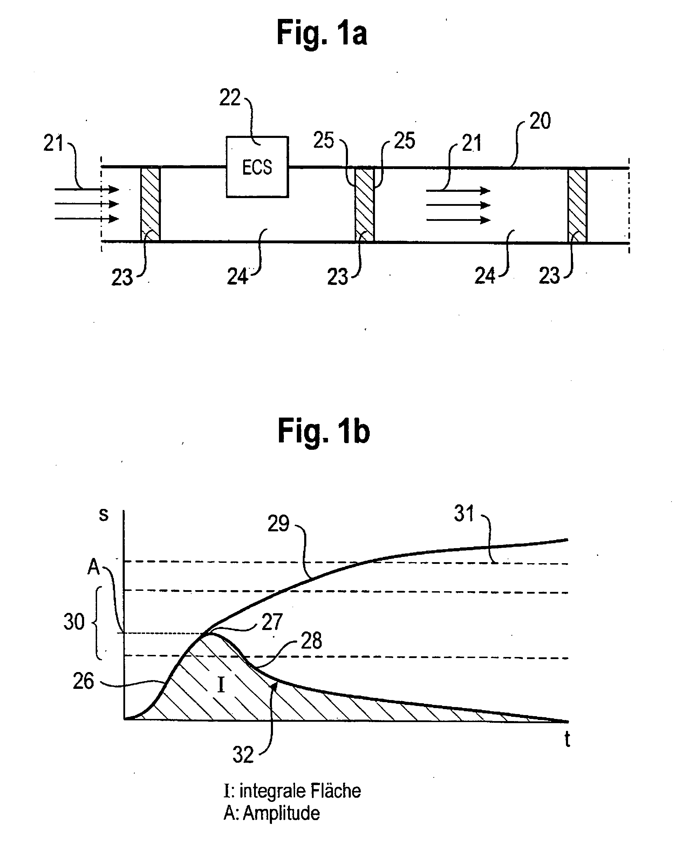

- Fig. 1a: zeigt schematisch eine Gasleitung, z. B. ein Messröhrchen 20 von z. B. 2 mm

Innendurchmesser eines erfindungsgemäßen Messgerätes, das von einem Gasstrom 21 durchströmt wird. In das Messröhrchen 20 ragt ein elektrochemischer Sensor (ECS) 22, der vom Gasstrom 21 beaufschlagt wird, so dass eine Messung initiiert wird. Der Gasstrom 21 weist erfindungsgemäß mindestens ein relativ kleines, definiertes Gasgemischvolumen 23 des zu detektierenden Gasgemisches auf, das in einem Trägergasvolumen 24 eingebettet ist und mitgenommen bzw. transportiert wird. Das Gasgemisch ist z. B. Biogas und das Trägergas z. B. Luft. Der Sensor 22 ist z. B. ein H₂S messender elektrochemischer Sensor.

Das definierte Gasgemischvolumen 23 wird von der Gasleitungswandung z. B. einer Messröhrchenwandung und dem Trägergasstromvolumen 24 eingegrenzt, wobei Gasphasengrenzen 25 zwischen dem Trägergasstrontvolumen 24 und dem Gasgemischvolumen 23 ausgebildet sind. Diese Gasphasengrenzen 25 sind in Wirklichkeit selbstverständlich nicht, wie abgebildet, symmetrisch ausgebildet, sondern verlaufen mehr oder weniger chaotisch. Gleichwohl wird erfindungsgemäß dafür Sorge getragen, dass sich ein separates abgegrenztes Gasgemischpolster 23 bzw. separates abgegrenztes Gasgemischvolumen 23 am Sensor 22 kurzzeitig vorbei bewegt und das Gasgemisch detektiert werden kann.

Fig. 1b zeit idealisiert eine von einem erfindungsgemäßen Gasmessgerät aufgrund der Sensorreaktion des elektrochemischen Sensors bildlich dargestellte Messwertkurve, z. B. eine H₂S-Messwertkurve 32 einer Messung eines Gasgemischpolsters 23 mit einer Ansprechphase 26, einer Amplitude 27 und einer Abklingphase 28. Des Weiteren zeigt Fig. 1b gestrichelt die Messwertkurve 29 des detektierten Gasgemisches, die sich ergäbe, wenn der Gasstrom 21 ausschließlich aus dem zu detektierenden Gasgemisch bestünde. In Fig. 1b sind auf der Abszisse die Messzeit t und auf der Ordinate die Konzentration s des zu bestimmenden Gasgemischbestandteils abgeteilt.

Des Weiteren sind in Fig. 1b ein Konzentrationsbereich 30, der einen optimalen Messbereich für lange Sensorstandzeiten zeigt, sowie die Messbereichsgrenze 31 des darunter befindlichen nominalen Messbereichs des Sensors eingezeichnet.

Fig. 1a und 1b zeigen beispielhaft, dass ein zwischen Trägergasvolumina. 24 abgegrenztes Gasgemischpolster 23 vorbestimmten Volumens, das mit einer vorbestimmten Geschwindigkeit am Sensor 22 vorbeiströmt und den Sensor 22 eine vorbestimmte Zeitspanne beaufschlagt, einen Abbruch der Reaktion des sensitiven Stoffes des Sensors bewirkt (Vergleich zwischen Kurve 32 und 29). Erfindungsgemäß wird somit der Sensor mit einem unverdünnten Gasgemischvolumen 23. beaufschlagt, das kleiner ist als das Gasgemischvolumen, das erforderlich wäre, um eine vollständige Messung gemäß Messwertkurve 29 auszuführen. Dadurch wird die Reaktion der sensitiven Stoffe des Sensors abgebrochen, wenn der Sensor noch nicht im z. B. überbelastenden Bereich, z. B. oberhalb oder Messwertgrenze 31 arbeitet, sondern in einem ausgewählten optimalen Bereich 30, in dem der Sensor störungsfrei sehr lange Zeit, z. B. zwei Jahre arbeiten kann.

Durch Eichung des Sensors bzw. des Messgerätes mit entsprechenden vorbekannten Trägergas/Messgasgemisch-. Polstertaktfolgen kann z. B. die Messung der Amplitude der Messwertkurve 32 des Messgerätes oder die Bestimmung einer bestimmten Fläche unterhalb der Messwertkurve 32 zur exakten Bestimmung der tatsächlichen Konzentration, basierend auf einer Rückrechnung, verwendet werden.

Besonders genaue Konzentrationsmessungen ergeben sich, wenn die Messung taktweise mit konstanten Gasgemischvolumina 23 wiederholt wird, und zwar mit einer konstanten Wiederholzeit bzw. Taktzeit, in der die Reaktion des Sensors 22 noch nicht nennenswert abklingt und sich die Messwerte noch im Bereich der Amplitude 27 der Messwertkurve 32 der vorhergehenden Messung befinden. Fig. 1a zeigt z. B. drei zeitlich getaktete, in den strömenden Trägergasstrom eingebrachte Messgasgemischpolster 23 bzw. -volumina 23, die nacheinander vom Trägergas am Sensor 22 vorbeigeschoben und vom Sensor 22 vorläufiger Konzentrationsmesswerte detektiert werden.

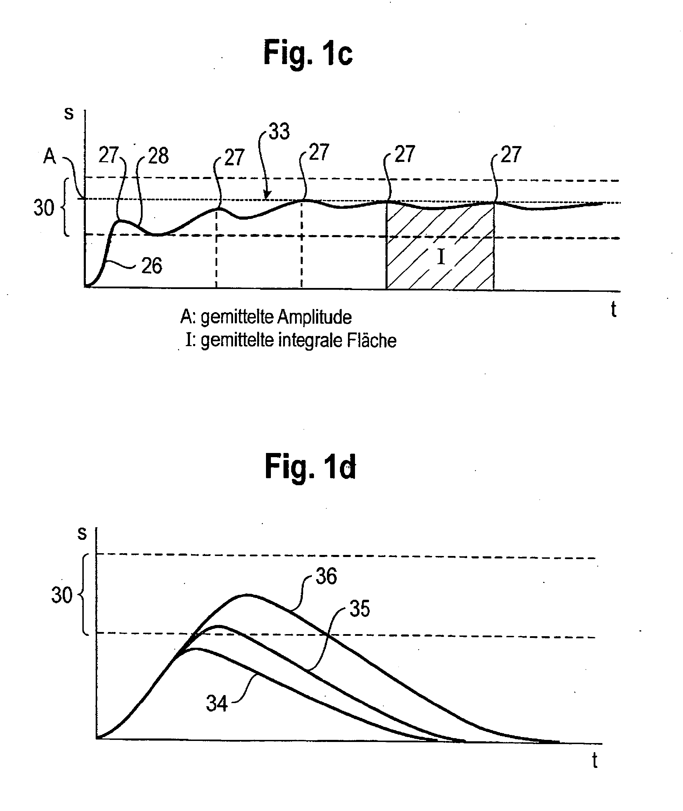

Fig. 1c stellt idealisiert eine Messwertkurve 33 dar, die sechs Pulse mit sechs identischen Messgasgemischpolstern 23 beinhaltet und demgemäß sechs Amplituden 27 andeutet, wobei der Sensor 22 mit sechs identischen Messgasgemischpolstern 23 bzw. Messgasgemischvolumina 23 (= Pulse) in konstanten Zeitabständen (= Takten) beaufschlagt wurde. Dabei ergibt sich eine relativ genaue gemittelte Amplitude A oder die Möglichkeit einer sehr genauen integralen gemittelten Flächenauswertung unterhalb der Messwertkurve 33 (z.B. Messfeld I).

Beispielsweise wird zur Konzentrationsmessung ein H₂S-Sensor, der im Bereich von 0 bis 100 ppm H₂S misst, mit jeweils identischem Messgasgemischvolumina 23 zwischen 5 und 250, insbesondere zwischen 50 und 200 mm³, die in jeweils identische Trägergasvolumina 24 zwischen 30 und 750, insbesondere 300 und 600 mm³ eingebettet sind, mit Taktzeiten zwischen 20 und 500 , insbesondere zwischen 100 und 30 msec beaufschlagt.

Die Erfindung sieht vor, die Konzentrationsmessung auf einen bestimmten, insbesondere optimalen, unterhalb der Messbereichsgrenze 31 liegenden Messbereich 30 zu begrenzen, der vorzugsweise im Bereich von 5 - 40 %, insbesondere zwischen 10 und 25 % des nominalen Messbereichs unterhalb der Bereichsgrenze 31 liegt. Dieser vorher durch Eichung festgelegte optimale Bereich 30 wird bei einer realen Messung im Einsatz des Messgerätes zunächst mit einem Messgasgemischvolumen 23, das einen unbekanntem Anteil an zu detektierendem Gasgemischbestandteil aufweist, angesteuert, indem der Sensor zunächst mit einem bestimmten, relativ geringen Gasgemisch- bzw. Polstervolumen 23 gepulst bzw. mehrmals getaktet beaufschlagt wird. Fällt die Messwertkurve bzw. fallen die Messwerte in den optimalen Messbereich 30, wird die Messung ohne weiteres fortgeführt und die Konzentration bestimmt. Fällt die Messwertkurve jedoch noch nicht in den Messbereich 30, wird z. B. schrittweise das Gasgemischvolumen 23 erhöht und damit auch die Zeit, während der der Sensor mit dem Messgasgemisch beaufschlagt wird, verlängert, bis die Amplitude der Messwertkurve im optimalen Messbereich 30 liegt. Wird dagegen der Messbereich bereits bei der ersten Messung überschritten, wird das Gasgemischvolumen 23 entsprechend verringert, oder das Messgasgemisch vor der Messung definiert verdünnt.

Anstelle der Erhöhung oder Verringerung der Gasgemischvolumina 23 oder in Kombination damit kann die Taktkzeit, d. h. die Wiederholzeit des Einbringens der Gasgemischvolumina 23 in den Trägergasstrom, z. B. ebenfalls schrittweise, variiert d. h. erhöht oder verringert werden. Eine dritte erfindungsgemäße Möglichkeit, den optimalen Messbereich anzusteuern, besteht darin, die Strömungsgeschwindigkeit des Gasstromes zu erhöhen oder zu erniedrigen.

Aufgrund der Eichung ist das Polstervolumen 23 des Messgasgemisches in Kombination mit bestimmten Taktzeiten und Gasströmungsgeschwindigkeiten, ein definiertes Maß für die tatsächliche Konzentration des zu detektierenden Gasgemischbestandteils im Messgasgemisch.

Fig. 1d verdeutlicht die Reaktion eines elektrochemischen Sensors bei einer Verlängerung der Taktzeit oder bei Vergrößerung der Gasgemischvolumina 23. Dabei entsprechen die dargestellten Messkurven 34, 35 und 36 identischen Konzentrationen des zu detektierenden Gasgemischbestandteils. Es wurde lediglich die Messdauer, d. h. die Dauer der Beaufschlagung des Sensors erhöht, woraus eine Anhebung der Messwerte in den optimalen Messbereich 30 resultiert. Wie oben beschrieben, kann die Messdauer durch die Variation der Gasgemischvolumina und/oder der Taktzeiten und/oder der Strömungsgeschwindigkeiten beeinflusst werden.

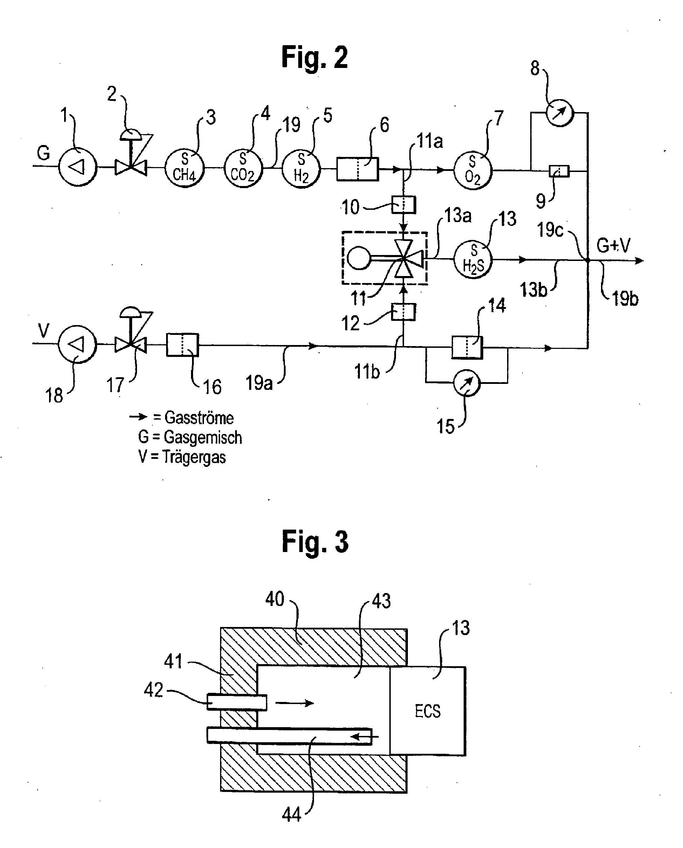

Ein erfindungsgemäßes Biogas-Messgerät mit eines elektrochemischen H₂S-Sensor kann gemäß Fig. 2 mit folgenden Bauteilen ausgerüstet sein.

In einer Messgasgemischleitung 19 sind - wie abgebildet - aufeinanderfolgend z. B. eine Gaspumpe 1, ein Druckregler 2, ein CH₄-Sensor 3, ein CO₂-Sensor 4, ein H₂-Sensor 5 und ein O₂-Sensor 7 integriert.

Schaltungstechnisch parallel ist eine Trägergasleitung 19a (z.B. Luftleitung) vorgesehen, in die - wie ebenfalls abgebildet - aufeinanderfolgend z. B. eine Gaspumpe 18 und ein Druckregler 17 integriert sind.

Zwischen beide Gasleitungen 19 und 19a ist ein Dreiwegeventil 11 geschaltet, das über eine Abzweigleitung 11a mit der Messgasgemischleitung 19 und über eine Abzweigleitung 11b mit der Trägergasleitung 19a in Verbindung steht. Zweckmäßigerweise zweigen die Leitungen 11a, 11b jeweils hinter den Druckreglern 2, 17 ab, wobei die Leitung 11a vorzugsweise hinter dem H₂-Sensor oder dem O₂-Sensor abzweigt. Die freien Enden der Gasleitungen 19, 19a münden an einem Gasleitungsknotenpunkt 19c in einer gemeinsamen Gasableitung 19b, die aus dem Messgerät herausführt.

Jeweils vor dem Gasleitungsknotenpunkt 19c sind zweckmäßigerweise in die Leitung 19 eine Drossel 9, die mit einem Drucksensor 8 überwacht wird und in die Leitung 19a eine Drossel 14, die von einem Drucksensor 15 überwacht wird, eingefügt. Des weiteren sind zweckmäßigerweise vor den Abzweigleitungen 11a, 11b je eine Drossel 6, 16 und in den Abzweigleitungen 11a, 11b je eine Drossel 10, 12 angeordnet. Vom Dreiwegeventil 11 führt eine Gasverbindungsleitung 13a zu einem elektrochemischen H₂S-Sensor 13 und von dort eine Gasverbindungsleitung 13b zum Gasleitungsknotenpunkt 19c.

Im Betrieb des erfindungsgemäßen Gasmessgerätes fließt druckgeregelt permanent, vorzugsweise gepumpt, ein erster Trägergasstrom, z. B. ein Luftstrom durch die Leitung 19a über den Knotenpunkt 19c durch die Gasableitung 19b sowie aus der Trägergasleitung 19a permanent abgezweigt in die Abzweigleitung 11b ein zweiter abgezweigter Trägergasstrom in einen Eingang das entsprechend geöffneten Dreiwegeventils 11 und durch das Ventil 11 hindurch in die Gasverbindungsleitung 13a, durch den H₂₋Sensor 13 und die Gasverbindungsleitung 13b und vereint sich im Knotenpunkt 19c mit dem ersten Hilfsgasstrom.

Zeitlich parallel fließt druckgeregelt und permanent, ebenfalls vorzugsweise gepumpt, ein Gasgemischstrom durch die Leitung 19 und soweit vorhanden, durch die integrierten Sensoren 3, 4, 5 und 7 über den Knotenpunkt 19c in die Gasableitung 19b, wobei über die Abzweigleitung 11a das Gasgemisch mit einem vorbestimmten Druck am Ventil 11 ansteht.

Die optionalen Drosseln 6 und 16 bestimmen den Volumenstrom der beiden Gase und führen zu von der Schaltstellung des Ventils 11 weitgehend unabhängigen Volumenströme. Durch die optionalen Drosseln 9 und 10 wird das Teilungsverhältnis des Messgasgemischstroms und durch die optionalen Drosseln 12 und 14 das Teilungsverhältnis des Trägergasstroms, bestimmt. Hierdurch sind die Volumenströme vom Messgasgemisch und vom inerten Trägergas durch den ECS 13 festgelegt. Die Drosseln sind konstant und das Teilungsverhältnis kann somit einmalig mit einem Testgas bestimmt werden.

Ein von der. Dichte abhängiger Einfluss des Gasgemischvolumenstroms kann durch eine nähere Bestimmung der Dichte aus den Sensorsignalen oder eine optionale Dichtemessung reduziert werden. Änderungen der Volumenströme des Gasgemisches und des Trägergases aufgrund von Druckschwankungen können durch die optionalen Drucksensoren 8 und 15 reduziert werden.

Als Dreiwegeventil 11 wird vorzugsweise ein handelsübliche direktwirkendes 3/2-Wege-Wippenmagnetventil für Pneumatik verwendet, das z. B. ein Polyamidgehäuse aufweist, in dem eine Gleichstromspule und ein Fluidgehäuse miteinander verbunden, z. B. verschraubt sind. Im Gehäuse bewegt sich ein kippbar gelagerter Anker ähnlich einer Wippe, der das Ventil schaltet. Die geringe Kippbewegung der Wippe ist verschleißfrei. Derartige Ventile arbeiten steuerbar und sehr schnell, so dass auch sehr kleine und variable Gasgemischvolumina 23 erzeugt und in den permanenten Trägergasstrom eingeschleust werden können, wie weiter unten noch erläutert wird.

Eine weitere Ausführungsform der Erfindung sieht vor, den H₂S-Sensor mit einer besonderen, eine weitgehend noch laminare Strömung gewährleistende Konstruktion einer Anströmmesskammer 43 zu .kombinieren, wie sie in Fig. 3 schematisch abgebildet ist. Dabei handelt es sich um einen z B. kreisrunden Hohizyiinderkörper 40 mit einer Bodenwandung 41, wobei in die Deckenwandung der ECS 13 eingebracht ist oder - wie abgebildet - die Deckenwandung bildet. Die Bodenwandung 41 weist eine auf den Sensor gerichtete Gaszuführleitung 42 auf, die lediglich die Bodenwandung 41 durchgreift oder nur ein kurzes Stück in die Kammer 43 ragt. Eine ebenfalls auf den Sensor gerichtete Abführleitung 41 durchgreift ebenfalls die Bodenwandung 41, ragt jedoch in den Innenraum der Kammer 43 bis kurz vor den Sensor 13. Eine derartige z. B. 1 cm³ große Kammer 43 stellt aufgrund ihres geringen Volumens und besonderen Ausgestaltung sowie der kurzen Verweilzeit des Gasgemischvolumens 23 in der Messkammer auf besonders einfache Weise sicher, dass in der Messkammer keine störende Gasvermischung zwischen dem Trägergas rund dem Gasgemisch stattfindet, was insbesondere in Kombination mit dem ausgewählten Wippenmagnetventil gewährleistet werden kann.

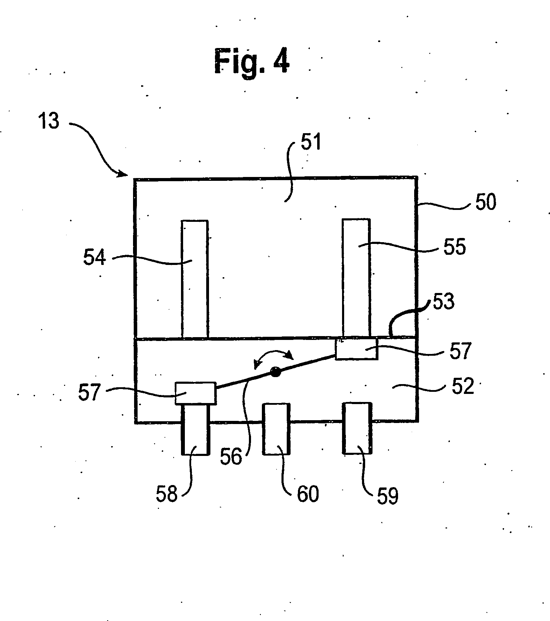

Fig. 4 zeigt schematisch die erfindungsgemäße Verwendung eines an sich bekannten 3/2-Wege-Wippenmagnetventils 13. Das Ventil 13 weist ein Gehäuse 50 auf, in dem eine relativ große Einrichtungskammer 51 von einer relativ kleinen Gaskammer 52 durch eine Wandung 53 gasdicht abgeteilt ist. In der Kammer 51 sind zwei zur Kammer 52 hin ausgerichtete Elektromagneten 54, 55 untergebracht, die mit elektrischen Leitungen (nicht dargestellt) in Verbindung stehen. Unterhalb der Wandung 53 ist eine doppelarmige Schaltwippe 56 auf- und abschwenkbar auf einer Drehachse in der Kammer 52 gelagert, die an ihren Enden mit Permanentmagneten ausgerüstet ist. Die Enden der Wippe 56 stehen außerdem mit Schließelementen 57 in Verbindung, die eine erste Gaszuleitung 58 und eine zweite Gaszuleitung 59 verschließen können, wobei die Gaszuleitungen 58 und 59 von außen in die Kammer 52 führen. Eine von der Wippe 56 unbeeinflusste Gasableitung 60 führt ebenfalls von außen in die Gaskammer 52.

Nach der Erfindung ist eine Gaszuleitung 59 mit einer Trägergaszuleitung, z. B. mit der Abzweigleitung 11b und die Gaszuleitung 58 mit einer Gasgemischzuleitüng, z. B. der Abzweigleitung 11a verbunden. Die Gasableitung 60 kann mit der Gasverbindungsleitung 13a in Verbindung stehen.

Im Betrieb fließt das Trägergas über die Gaszuleitung 59 in die Gaskammer 52 und über-die Gasableitung 60 aus der Gaskammer 52. Zum Pulsen bzw. Takten wird die Wippe 56 so geschaltet, dass die Gaszuleitung 59 geschlossen und die Gaszuleitung 58 geöffnet wird.

Anstelle eines 3/2-Wege-Wippenmagnetventils kann erfindungsgemäß auch ein 2/2-Wege-Gasventil verwendet werden. Dabei kann vorzugsweise die Abzweigung des Trägergasteilstroms 11b entfallen und das Gasgemischvolumen 23 direkt in den Trägergasstrom 19a geleitet werden. Gleichermaßen kann erfindungsgemäß der Gasgemischteilstrom 11a entfallen und ein Gasgemischvolumen 23 direkt aus der Hauptströmung 19 des Gasgemisches entnommen und in den Trägergasstrom 19a geleitet werden. Für diese Variante des erfindungsgemäßen Verfahrens sind die Hauptströme 19 und 19a direkt an das 3/2- oder 2/2-Wege-Ventil angeschlossen.

Ein erfindungsgemäßes Messgerät steht mit einer Steuer- und Auswertevorrichtung mit z. B. elektronischen Komponenten (nicht dargestellt) für vorzugsweise alle Elemente des Messgerätes wie Pumpen, Drosseln, Ventile, Kontrolleinrichtungen und Auswerteeinrichtungen für die Sensoren in Verbindung. Zumindest weist die Steuervorrichtung eine Steuereinrichtung für das Ventil 11 auf, die mit einer der Erfindung entsprechenden die Rechenoperationen ausführenden Software arbeitet.

Das erfindungsgemäße Verfahren ermöglicht nebenbei auch die Regeneration und die Konditionierung des Sensors 13, z. B. eines H₂S-Sensors, indem dem Trägergas insbesondere der Luft, zumindest zeitweise Bestandteile beigemengt werden, die der Sensor nach Reaktionen mit dem Messgasgemischbestandteil entbehrt. Es wird beispielsweise Wasser in Gasphase beigemengt. Durch diese Maßnahme wird die Lebensdauer bzw. die Funktionsdauer des Sensors, z. B. des H₂S-Sensors, ebenfalls erhöht, die hauptsächlich aber dadurch erhöht wird, dass der Sensor mit dem erfindungsgemäßen Pulsverfahren bzw. Taktverfahren mit unverdünnten Gasgemischvolumen immer in einem optimalen Messbereich betrieben wird.

## Patentansprüche

1. Verfahren zum Messen der Konzentration eines Gasgemischbestandteils in einem strömenden Gasgemisch, insbesondere zur Ermittlung des H₂S-Gehalts in einem Biogas unter Verwendung eines den Gasgemischbestandteil detektierenden elektrochemischen Sensors (ECS) mit folgenden Verfahrensschritten:
a) Vorhalten eines zu detektierenden, vorzugsweise statischen Gasgemischvolumens unter vorbestimmtem Druck,
b) Erzeugung eines vorzugsweise permanenten Trägergasstroms aus einem den zu detektierenden Gasgemischbestandteil nicht enthaltenden Trägergas,
c) Erzeugen einer Gasartenströmung in einer Gasleitung mit wechselnden durch Phasengrenzen abgegrenzten, separaten Gasartenvolumina durch Einbringen mindestens eines Gasgemischvolumens vorbestimmter Größe in den Trägergasstrom,
d) Beaufschlagung des elektrochemischen Sensors mit der Gasartenströmung, wobei das Gasgemisch mit vorbestimmter Geschwindigkeit am Sensor vorbeiströmt und den Sensor eine vorbestimmte Zeitspanne beaufschlagt, so dass lediglich mindestens ein vorläufiger Messwert, der unterhalb des der tatsächlichen Konzentration des Gasgemischbestandteils entsprechenden Messwertes liegt, ermittelt wird,
e) Verwendung des vorläufigen Messwertes zur Berechnung der tatsächlichen Konzentration des Gasgemischbestandteils in einer Messwertumrechnungseinrichtung, wobei die tatsächliche Konzentration des Gasgemischbestandteils durch Rückrechnung aufgrund von Eichwerten ermittelt wird.

2. Verfahren nach Anspruch 1,
**dadurch gekennzeichnet , dass** ein vorzugsweise permanenter Gasgemischstrom erzeugt und aus dem Gasgemischstrom das zu detektierende Gasgemischvolumen abgezweigt wird.

3. Verfahren nach Anspruch 1 und/oder 2,
**dadurch gekennzeichnet , dass** ein permanenter abgezweigter Trägergasstrom aus dem Trägergasstrom erzeugt und in diesen das Gasgemischvolumen eingebracht wird.

4. Verfahren nach einem oder mehreren der Ansprüche 1 bis 3,
**dadurch gekennzeichnet , dass** das Einbringen getaktet über ein Ventil erfolgt, das mit dem Trägergasstrom und dem Gasgemischvolumen in Verbindung steht und das mit einer Steuereinrichtung gesteuert geschaltet wird.

5. Verfahren nach Anspruch 4,
**dadurch gekennzeichnet , dass** vor dem Takten im Ventil das statische Gasgemischvolumen ansteht und das Ventil permanent vom Trägergasstrom durchströmt wird.

6. Verfahren nach Anspruch 5,
**dadurch gekennzeichnet** , das s der Trägergasstrom mit einer entsprechenden Ventilschaltung kurzzeitig gestoppt und dann durch entsprechendes Öffnen des Ventils das Gasgemischvolumen erzeugt und in den Trägergasstrom eingefügt wird.

7. Verfahren nach Anspruch 5,
**dadurch gekennzeichnet , dass** das Gasgemischvolumen durch entsprechendes Öffnen des Ventils in den permanent strömenden Trägergasstrom eingefügt wird.

8. Verfahren nach einem oder mehreren der Ansprüche 4 bis 7,
**dadurch gekennzeichnet , dass** ein 3/2-Wege-Wippenmagnetventil verwendet wird.

9. Verfahren nach einem oder mehreren der Ansprüche 4 bis 7,
**dadurch gekennzeichnet , dass** mehrmals mit gleicher Taktzeit und gleicher Öffnungsdauer des Ventils für das Gasgemisch ein Gasartenstrom in der Gasleitung erzeugt wird, der mehrere wechselnde Volumina der beiden Gasarten Trägergas und Gasgemisch aufweist und dass mit diesem Gasartenstrom der Sensor beaufschlagt wird, wobei die Taktzeit und Öffnungsdauer so gewählt sind, dass die Reaktion des Sensors keine Zeit hat, deutlich abzuklingen und der Sensor permanent nahezu gleichbleibende Messwerte im Amplitudenbereich ermittelt.

10. Verfahren nach Anspruch 9,
**dadurch gekennzeichnet , dass** ein mehreren Amplitudenmesswerten entsprechender gemittelter Messwert oder ein integraler Flächenwert unter einer Messwertekurve für die Rückrechnung verwendet werden.

11. Verfahren nach einem oder mehreren der Ansprüche 1 bis10,
**dadurch gekennzeichnet , dass** Gasgemischvolumina erzeugt werden in der Größenordnung von 5 bis 250 insbesondere von 50 bis 200 mm³.

12. Verfahren nach einem oder mehreren der Ansprüche 1 bis 11,
**dadurch gekennzeichnet , dass** Trägergasvolumina zwischen den Gasgemischvolumina erzeugt werden in der Größenordnung von 30 bis 750, insbesondere von 300 bis 600 mm³.

13. Verfahren nach einem oder mehreren der Ansprüche 1 bis 12,
**dadurch gekennzeichnet , dass** Taktzeiten zwischen den Gasgemischvolumina von 20 bis 500, insbesondere von 100 bis 300 msec verwendet werden.

14. Verfahren nach einem oder mehreren der Ansprüche 1 bis 13,
**dadurch gekennzeichnet , dass** insbesondere im Falle der Messung von H₂S-Konzentrationen Luft als Trägergas verwendet wird.

15. Verfahren nach einem oder mehreren der Ansprüche 1 bis 14,
**dadurch gekennzeichnet , dass** z. B im Falle zu hoher Konzentrationen des Gasgemischbestandteils im Gasgemisch eine vor der Messung definierte verdünnung des Gasgemisches erzeugt wird und die Gasgemischvolumina aus diesem verdünnten Gasgemisch erzeugt werden.

16. Verfahren nach einem oder mehreren der Ansprüche 1 bis 15,
**dadurch gekennzeichnet , dass** der Sensor in einem Bereich von 5 - 40 %, insbesondere zwischen 10 und 25 % des nominalen Messbereichs betrieben wird.

17. Vorrichtung zum Messen der Konzentration eines Gasgemischbestandteils in einem Gasgemisch, insbesondere zur Ermittlung des H₂S-Gehalts in einem Biogas, mit einem den Gasgemischbestandteil detektierenden elektrochemischen Sensor, ausgebildet zur Durchführung des Verfahrens nach einem oder mehreren der Ansprüche 1 bis 16, aufweisend
- eine Messgasgemischleitung (19),
- eine Trägergasleitung (19a),
- ein schaltbares Mehrwegeventil (11) als Mittel zum Erzeugen einer Gasartenströmung in einer Gasleitung kit wechselnden, durch Phasengrenzen abgegrenzten,
- separaten Gasartenvolumina durch Einbringen mindestens eines Gasgemischvolumens vorbestimmter Größe in einen Trägergasstrom,
- einen elektrochemischen Sensor (13),
- Mittel zur Beaufschlagung des elektrochemischen Sensors (13) mit der Gasartenströmung, wobei das Gasgemisch mit vorbestimmter Geschwindigkeit am Sensor (13) vorbeiströmt und den Sensor (13) eine vorbestimmte Zeitspanne beaufschlagt, so dass lediglich mindestens ein vorläufiger Messwert, der unterhalb des der tatsächlichen Konzentration des Gasgemischbestandteils entsprechenden Messwertes liegt, ermittelt wird,
- Mittel zur Verwendung des vorläufigen Messwertes zur Berechnung der tatsächlichen Konzentration des Gasgemischbestandteils in einer Messwertumrechnungseinrichtung, wobei die tatsächliche Konzentration des Gasgemischbestandteils durch Rückrechnung aufgrund von Eichwerten ermittelt wird
- eine erste und eine zweite Gasverbindungsleitung (13a, 13b),
- eine den Sensor enthaltende Einrichtung (43), wobei die Messgasgemischleitung (19) und die Trägergasleitung (19a) je in einem Eingang des Mehrwegeventils (11) münden und ein Ausgang des Mehrwegeventils (11) über die erste Gasverbindungsleitung (13a) mit der den Sensor (13) aufweisenden Einrichtung (43) in Verbindung steht, von der die zweite Gasverbindungsleitung (13b) abgeht.

18. Vorrichtung nach Anspruch 17,
**dadurch gekennzeichnet dass** die Messgasgemischleitung (19) über eine Abzweigleitung (11a) und die Trägergasleitung (19a) über eine Abzweigleitung (11b) in das Mehrwegeventil (11) münden.

19. Vorrichtung nach einem oder mehreren der Ansprüche 17 bis 18,
**dadurch gekennzeichnet , dass** in der Messgasgemischleitung (19) vor der Abzweigleitung (11a) eine Drossel (6) und in der Abzweigleitung (11a) eine Drossel (10) angeordnet sind.

20. Vorrichtung nach einem oder mehreren der Ansprüche 17 bis 19,
**dadurch gekennzeichnet , dass** in der Trägergasleitung (19a) vor der Abzweigleitung (11b) eine Drossel (16) und in der Abzweigleitung (11b) eine Drossel (12) angeordnet sind.

21. Vorrichtung nach einem oder mehreren der Ansprüche 17 bis 20,
**dadurch gekennzeichnet , dass** die Messgasgemischleitung (19) und die Trägergasleitung (19a) hinter den Abzweigungen der Abzweigungsleitungen (11a, 11b) in einem Knotenpunkt (19c) zusammenkommen und vom Knotenpunkt (19c) eine Gasableitung (19b) aus der Vorrichtung führt, wobei vorzugsweise auch die Gasverbindungsleitung (13b) zum Knotenpunkt (19c) geführt ist.

22. Vorrichtung nach einem oder mehreren der Ansprüche 17 bis 21,
**dadurch gekennzeichnet, dass** in der Messgasgemischleitung (19) und vorzugsweise auch in der Trägergasleitung (19a) eine Gaspumpe (1, 18) angeordnet ist.

23. Vorrichtung nach Anspruch 22,
**dadurch gekennzeichnet , dass** den Pumpen (1, 18) jeweils ein Druckregler (2, 17) nachgeordnet ist.

24. Vorrichtung nach einem oder mehreren der Ansprüche 17 bis 23,
**dadurch gekennzeichnet , dass** nach der Abzweigung in der Messgasgemischleitung (19) eine mit einem Drucksensor (8) überwachte Drossel (9) angeordnet ist.

25. Vorrichtung nach einem oder mehreren der Ansprüche 17 bis 24,
**dadurch gekennzeichnet , dass** nach der Abzweigung in der Trägergasleitung (19a) eine mit einem Drucksensor (15) überwachte Drossel (14) angeordnet ist.

26. Vorrichtung nach einem oder mehreren der Ansprüche 17 bis 25,
**dadurch gekennzeichnet , dass** die den Sensor (13) aufweisende Einrichtung eine zweckmäßigerweise kreiszylindrische Anströmmesskammer (43) aufweist, die von einer Bodenwandung (41) und einer Deckenwandung begrenzt wird, wobei in der Deckenwandung der Sensor (13) in die Kammer ragend positioniert ist und die Bodenwandung (41) eine auf den Sensor (13) gerichtete, lediglich in die Kammer führende Gaszuführleitung (42) und eine ebenfalls auf den Sensor (13) gerichtete und bis kurz vor den Sensor (13) reichende Gasabführleitung (41) aufweist und wobei die Gaszuführleitung (42) mit der Gasverbindungsleitung (13a) und die Gasabführleitung (41) mit der Gasverbindungsleitung (13b) verbunden sind.

27. Vorrichtung nach einem oder mehreren der Ansprüche 17 bis 26,
**dadurch gekennzeichnet , dass** ein 3/2-Wege-Wippenmagnetventil (11) verwendet wird.

28. Vorrichtung nach Anspruch 27,
**dadurch gekennzeichnet , dass** die Gasverbindungsleitung (13a) mit einer Gasableitung (60) des Wippenmagnetventils (11) und die Abzweigleitungen (11a, 11b) mit je einer Gaszuleitung (58, 59) des Wippenmagnetventils (11) verbunden sind, wobei die Gaszuleitungen (58, 59) von einer doppelarmigen Schaltwippe (56) wechselseitig verschließbar sind.

29. Vorrichtung nach einem oder mehreren der Ansprüche 17 bis 28,
**dadurch gekennzeichnet , dass** in der Messgasgemischleitung (19) weitere Gassensoren (3, 4, 5, 7) angeordnet sind.

30. Vorrichtung nach Anspruch 29,
**dadurch gekennzeichnet , dass** die weiteren Gassensoren zwischen dem Druckregler (2) und der Drossel (6) angeordnet sind.

## Claims

1. A method of measuring the concentration of a gas mixture constituent in a flowing gas mixture, in particular for determining the H₂S content in a biogas, using an electrochemical sensor (ECS) which detects the gas mixture constituent, having the following method steps:
a. Provision under a predetermined pressure of a, preferably static, gas mixture volume to be detected,
b. Production of a, preferably permanent, carrier gas stream from a carrier gas not containing the gas mixture constituent to be detected,
c. Production in a gas line of a gas types flow with alternating separate gas types volumes, defined by phase interfaces, through the introduction of at least one gas mixture volume of pre-determined magnitude into the carrier gas stream,
d. Action of the gas types flow upon the electrochemical sensor, wherein the gas mixture flows past the sensor at a pre-determined speed and acts upon the sensor for a pre-determined period of time, so that merely at least a preliminary measured value, lying below the measured value corresponding to the actual concentration of the gas mixture constituent, is determined,
e. Use of the preliminary measured value to calculate the actual concentration of the gas mixture constituent in a measured value conversion device, wherein the actual concentration of the gas mixture constituent is determined through back calculation on the basis of calibration values.

2. A method according to claim 1, **characterised in that** a, preferably permanent, gas mixture stream is produced and the gas mixture volume to be detected is diverted from the gas mixture stream.

3. A method according to claim 1 and/or 2, **characterised in that** a permanent diverted carrier gas stream is produced from the carrier gas stream and the gas mixture volume is introduced therein.

4. A method according to one or more of claims 1 to 3, **characterised in that** the introduction takes place in a clocked manner via a valve which is connected to the carrier gas stream and the gas mixture volume and which is switched in a controlled manner by means of a control device.

5. A method according to claim 4, **characterised in that** the static gas mixture volume is present in the valve before the clocking and the carrier gas stream flows through the valve in a permanent manner.

6. A method according to claims 5, **characterised in that** the carrier gas stream is temporarily stopped by means of an appropriate valve switching and the gas mixture volume is then produced through appropriate opening of the valve and is inserted into the carrier gas stream.

7. A method according to claim 5, **characterised in that** the gas mixture volume is inserted into the permanently flowing carrier gas stream through appropriate opening of the valve.

8. A method according to one or more of claims 4 to 7, **characterised in that** a 3/2 way rocker solenoid valve is used.

9. A method according to one or more of claims 4 to 7, **characterised in that** with the same clock time and the same opening duration of the valve for the gas mixture, a gas types stream is repeatedly produced in the gas line, which gas types stream has a plurality of alternating volumes of the two gas types which are carrier gas and gas mixture, and **in that** the sensor is acted upon by this gas types stream, wherein the clock time and opening duration are selected in such a manner that the reaction of the sensor does not have time to clearly subside and the sensor permanently determines virtually constant measured values in the amplitude range.

10. A method according to claim 9, **characterised in that** an averaged measured value corresponding to a plurality of amplitude measured values, or an integral area value under a measured values curve are used for the back calculation.

11. A method according to one or more of claims 1 to 10, **characterised in that** gas mixture volumes in the order of magnitude of 5 to 250 mm³, in particular 50 to 200 mm³, are produced.

12. A method according to one or more of claims 1 to 11, **characterised in that** carrier gas volumes in the order of magnitude of 30 to 750 mm³, in particular 300 to 600 mm³, are produced between the gas mixture volumes.

13. A method according to one or more of claims 1 to 12, **characterised in that** clock times between the gas mixture volumes of 20 to 500 msec, in particular 100 to 300 msec, are used.

14. A method according to one or more of claims 1 to 13, **characterised in that** air is used as the carrier gas, in particular in the event of measurement of H₂S concentrations.

15. A method according to one or more of claims 1 to 14, **characterised in that** a rarefaction, defined before measurement, of the gas mixture is produced e.g. in the event of excessively high concentrations of the gas mixture constituent in the gas mixture and the gas mixture volumes are produced from this rarefied gas mixture.

16. A method according to one or more of claims 1 to 15, **characterised in that** the sensor is operated in a range of 5 - 40%, in particular between 10 and 25%, of the nominal measuring range.

17. A device for measuring the concentration of a gas mixture constituent in a gas mixture, in particular for determining the H₂S content in a biogas, with an electrochemical sensor detecting the gas mixture constituent, designed to carry out the method according to one or more of claims 1 to 16, having
- a measuring gas mixture line (19),
- a carrier gas line (19a),
- a switchable multiple-way valve (11) as a means to produce in a gas line a gas types flow with alternating separate gas types volumes, defined by phase interfaces, through the introduction of at least one gas mixture volume of pre-determined magnitude into a carrier gas stream,
- an electrochemical sensor (13),
- means to act upon the electrochemical sensor (13) with the gas types flow, wherein the gas mixture flows past the sensor (13) at a pre-determined speed and acts upon the sensor (13) for a pre-determined period of time, so that merely at least a preliminary measured value, lying below the measured value corresponding to the actual concentration of the gas mixture constituent, is determined,
- means for using the preliminary measured value to calculate the actual concentration of the gas mixture constituent in a measured value conversion device, wherein the actual concentration of the gas mixture constituent is determined through back calculation on the basis of calibration values,
- a first and a second gas connecting line (13a, 13b),
- a device (43) containing the sensor, wherein the measuring gas mixture line (19) and the carrier gas line (19a) each issue in an inlet of the multiple-way valve (11) and an outlet of the multiple-way valve (11) is connected to the device (43), with the sensor (13), via the first gas connecting line (13a), from which device (43) there leads the second gas connecting line (13b).

18. A device according to claim 17, **characterised in that** the measuring gas mixture line (19) opens into the multiple-way valve (11) via a branch line (11a) and the carrier gas line (19a) opens into the multiple-way valve (11) via a branch fine (11b).

19. A device according to one or more of claims 17 to 18, **characterised in that** a throttle (6) is arranged in the measuring gas mixture line (19) so as to be before the branch line (11a) and a throttle (10) is arranged in the branch line (11 a).

20. A device according to one or more of claims 17 to 19, **characterised in that** a throttle (16) is arranged in the carrier gas line (19a) so as to be before the branch line (11 b) and a throttle (12) is arranged in the branch line (11b).

21. A device according to one or more of claims 17 to 20, **characterised in that** the measuring gas mixture line (19) and the carrier gas line (19a) meet at a junction (19c) after the branches of the branch lines (11a, 11b) and a gas discharge line (19b) runs out of the device from the junction (19c), wherein the gas connecting line (13b) preferably also runs to the junction (19c).

22. A device according to one or more of claims 17 to 21, **characterised in that** a gas pump (1, 18) is arranged in the measuring gas mixture line (19) and preferably also in the carrier gas line (19a).

23. A device according to claim 22, **characterised in that** in each case a pressure regulator (2, 17) is arranged downstream of the pumps (1, 18).

24. A device according to one or more of claims 17 to 23, **characterised in that** after the branch in the measuring gas mixture line (19) there is arranged a throttle (9) monitored by a pressure sensor (8).

25. A device according to one or more of claims 17 to 24, **characterised in that** after the branch in the carrier gas line (19a) there is arranged a throttle (14) monitored by a pressure sensor (15).

26. A device according to one or more of claims 17 to 25, **characterised in that** the device having the sensor (13) has an advantageously circular-cylindrical inflow measuring chamber (43) bounded by a bottom wall (41) and a cover wall, wherein the sensor (13) is positioned in the cover wall so as to project into the chamber and the bottom wall (41) has a gas feed line (42), directed at the sensor (13) and merely running into the chamber, and a gas discharge line (41), likewise directed at the sensor (13) and extending to just in front of the sensor (13), and wherein the gas feed line (42) is connected to the gas connecting line (13a) and the gas discharge line (41) is connected to the gas connecting line (13b).

27. A device according to one or more of claims 17 to 26, **characterised in that** a 3/2 way rocker solenoid valve (11) is used.

28. A device according to claim 27, **characterised in that** the gas connecting line (13a) is connected to a gas discharge line (60) of the rocker solenoid valve (11) and the branch lines (11a, 11b) are connected to a respective gas feed line (58, 59) of the rocker solenoid valve, wherein the gas feed lines (58, 59) are alternately closable by a two-armed rocker switch (56).

29. A device according to one or more of claims 17 to 28, **characterised in that** further gas sensors (3, 4, 5, 7) are arranged in the measuring gas mixture line (19).

30. A device according to claim 29, **characterised in that** the further gas sensors are arranged between the pressure regulator (2) and the throttle (6).

## Revendications

1. Procédé de mesure de la concentration d'un composant d'un mélange de gaz dans un mélange de gaz en circulation, en particulier pour la détermination de la teneur en H₂S dans un biogaz, en utilisant un capteur électrochimique (CEC) détectant le composant du mélange de gaz, comprenant les étapes de procédé suivantes consistant à :
a) maintenir en réserve un volume de mélange de gaz à détecter, de préférence statique, sous une pression prédéterminée,
b) produire un flux de gaz support de préférence permanent à partir d'un gaz support ne contenant pas le composant du mélange de gaz à détecter,
c) produire un flux d'un type de gaz dans une conduite de gaz avec des volumes de type de gaz séparés, changeants, délimités par des limites de phases, par introduction d'au moins un volume de mélange de gaz de taille prédéterminée dans le flux de gaz support,
d) soumettre le capteur électrochimique au flux du type de gaz, dans lequel le mélange de gaz passe devant le capteur à une vitesse prédéterminée et expose le capteur pendant une durée prédéterminée, de sorte qu'au moins une valeur de mesure provisoire qui se situe en dessous de la valeur de mesure correspondant à la concentration réelle soit uniquement déterminée,
e) utiliser de la valeur de mesure provisoire pour le calcul de la concentration réelle du composant de mélange de gaz dans un dispositif de conversion de la valeur de mesure, dans lequel la concentration réelle du composant de mélange de gaz est déterminée par calcul rétrograde sur la base de valeurs d'étalonnage.

2. Procédé selon la revendication 1, **caractérisé en ce qu'**un flux de mélange de gaz de préférence permanent est produit et **en ce que** le volume de mélange de gaz à détecter est dérivé du flux de mélange de gaz.

3. Procédé selon la revendication 1 et/ou 2. **caractérisé en ce qu'**un flux de gaz support permanent dérivé est produit à partir du flux de gaz support et que le volume de mélange de gaz est introduit dans celui-ci.

4. Procédé selon l'une quelconque ou plusieurs des revendications 1 à 3, **caractérisé en ce que** l'introduction se fait en cadence par l'intermédiaire d'une vanne, qui est en relation avec le flux de gaz support et le volume de mélange de gaz et qui est commandée par l'intermédiaire d'un circuit avec un dispositif de commande.

5. Procédé selon la revendication 4, **caractérisé en ce que** le volume de mélange de gaz statique s'accumule dans la vanne avant le cadencement et la vanne est traversée en permanence par le flux de gaz support.

6. Procédé selon la revendication 5, **caractérisé en ce que** le flux de gaz support est arrêté brièvement par un circuit de vanne correspondant et qu'ensuite, par une ouverture correspondante de la vanne, le volume de mélange de gaz est produit et est introduit dans le flux de gaz support.

7. Procédé selon la revendication 5, **caractérisé en ce que** le volume de mélange de gaz est introduit dans le flux de gaz support circulant en permanence par une ouverture correspondante de la vanne.

8. Procédé selon l'une quelconque ou plusieurs des revendications 4 à 7, **caractérisé en ce que** l'on utilise une électrovanne à bascule 3/2 voies.

9. Procédé selon l'une quelconque ou plusieurs des revendications 4 à 7, **caractérisé en ce que** l'on produit plusieurs fois avec la même cadence et la même durée d'ouverture de la vanne pour le mélange de gaz un flux de type de gaz dans la conduite de gaz qui présente plusieurs volumes changeants des deux types de gaz, gaz support et mélange de gaz, et **en ce que** le capteur est soumis à ce flux de type de gaz, dans lequel la cadence et la durée d'ouverture sont choisies de telle sorte que la réaction du capteur n'a pas le temps de s'éteindre nettement et que le capteur détecte en permanence des valeurs de mesure presque constantes dans la plage d'amplitudes.

10. Procédé selon la revendication 9, **caractérisé en ce que** l'on utilise une valeur de mesure dont la moyenne a été faite et qui correspond à plusieurs valeurs de mesure d'amplitude ou une valeur d'aire intégrale en dessous d'une courbe de valeurs de mesure pour le calcul rétrograde.

11. Procédé selon l'une quelconque ou plusieurs des revendications 1 à 10, **caractérisé en ce que** l'on produit des volumes de mélange de gaz de l'ordre de grandeur allant de 5 à 250, en particulier allant de 50 à 200 mm³.

12. Procédé selon l'une quelconque ou plusieurs des revendications 1 à 11, **caractérisé en ce que** l'on produit des volumes de gaz support entre les volumes de mélange de gaz de l'ordre de grandeur allant de 30 à 750, en particulier allant de 300 à 600 mm³.

13. Procédé selon l'une quelconque ou plusieurs des revendications 1 à 12, **caractérisé en ce que** l'on utilise des cadences entre les volumes de mélange de gaz allant de 20 à 500, en particulier allant de 100 à 300 msec.

14. Procédé selon l'une quelconque ou plusieurs des revendications 1 à 13, **caractérisé en ce que** l'on utilise l'air comme gaz support, en particulier dans le cas de la mesure des concentrations en H₂S.

15. Procédé selon l'une quelconque ou plusieurs des revendications 1 à 14, **caractérisé en ce que** l'on produit une dilution du mélange de gaz définie avant la mesure, par exemple dans le cas de concentrations trop élevées en composant du mélange de gaz, et que l'on produit les volumes de mélange de gaz à partir de ce mélange de gaz dilué.

16. Procédé selon l'une quelconque ou plusieurs des revendications 1 à 15, **caractérisé en ce que** le capteur fonctionne dans une plage allant de 5 à 40 %, en particulier entre 10 et 25 % de la plage de mesures nominales.

17. Dispositif de mesure de la concentration d'un composant d'un mélange de gaz dans un mélange de gaz, en particulier pour la détermination de la teneur en H₂S dans un biogaz, avec un capteur électrochimique détectant le composant du mélange de gaz, conçu pour réaliser le procédé selon l'une quelconque ou plusieurs des revendications 1 à 16, comprenant
- une conduite de mélange de gaz de mesure (19),
- une conduite de gaz support (19a),
- une vanne multi-voies commutable (11) comme moyen pour produire un flux de type de gaz dans une conduite de gaz, avec des volumes de type de gaz changeants, séparés, délimités par des limites de phase, par introduction d'au moins un volume de mélange de gaz de taille prédéterminée dans un flux de gaz support,
- un capteur électrochimique (13),
- un moyen pour soumettre le capteur électrochimique (13) au flux de type de gaz, dans lequel le mélange de gaz passe devant le capteur (13) à une vitesse prédéterminée et expose le capteur (13) pendant un laps de temps prédéterminé, de sorte qu'au moins une valeur de mesure provisoire, qui se situe en dessous de la valeur de mesure correspondant à la concentration réelle du composant de mélange de gaz, soit seulement déterminée,
- un moyen pour utiliser la valeur de mesure provisoire pour calculer la concentration réelle du composant de mélange de gaz dans un système de conversion des valeurs de mesure, dans lequel la concentration réelle du composant de mélange de gaz est déterminée par calcul rétrograde sur la base de valeurs d'étalonnage,
- une première et une deuxième conduite de raccordement de gaz (13a, 13b),
- un dispositif (43) comprenant le capteur,
dans lequel la conduite de mélange de gaz de mesure (19) et la conduite de gaz support (19a) débouchent chacune dans une entrée de la vanne multi-voies (11) et une sortie de la vanne multi-voies (11) est en relation avec le dispositif (43) comprenant le capteur (13) par l'intermédiaire de la première conduite de raccordement de gaz (13a), à partir de laquelle part la deuxième conduite de raccordement de gaz (13b).

18. Dispositif selon la revendication 17, **caractérisé en ce que** la conduite de mélange de gaz de mesure (19) débouche dans la vanne multi-voies (11) par l'intermédiaire d'une conduite de dérivation (11a) et la conduite de gaz support (19a) par l'intermédiaire d'une conduite de dérivation (11b).

19. Dispositif selon l'une quelconque ou plusieurs des revendications 17 à 18, **caractérisé en ce que**, dans la conduite de mélange de gaz de mesure (19), un étranglement (6) est disposé avant la conduite de dérivation (11a) et un étranglement (10) est disposé dans la conduite de dérivation (11a).

20. Dispositif selon l'une quelconque ou plusieurs des revendications 17 à 19, **caractérisé en ce que**, dans la conduite de mélange de gaz de mesure (19a), un étranglement (16) est disposé avant la conduite de dérivation (11b) et un étranglement (12) est disposé dans la conduite de dérivation (11b).

21. Dispositif selon l'une quelconque ou plusieurs des revendications 17 à 20, **caractérisé en ce que** la conduite de mélange de gaz de mesure (19) et la conduite de gaz support (19a) se rencontrent après les dérivations des conduites de dérivation (11a, 11b) en un point de rencontre (19c), et **en ce qu'**une conduite d'évacuation de gaz (19b) conduit hors du dispositif à partir du point de rencontre (19c), dans lequel la conduite de raccordement de gaz (13b) conduit de préférence également au point de rencontre (19c).

22. Dispositif selon l'une quelconque ou plusieurs des revendications 17 à 21, **caractérisé en ce qu'**une pompe de gaz (1, 18) est disposée dans la conduite de mélange de gaz de mesure (19) et de préférence également dans la conduite de gaz support (19a).

23. Dispositif selon la revendication 22, **caractérisé en ce qu'**un régulateur de pression (2, 17) est disposé respectivement après chaque pompe (1, 18).

24. Dispositif selon l'une quelconque ou plusieurs des revendications 17 à 23, **caractérisé en ce qu'**un étranglement (9), contrôlé par un capteur de pression (8), est disposé après la dérivation dans la conduite de mélange de gaz de mesure (19).

25. Dispositif selon l'une quelconque ou plusieurs des revendications 17 à 24, **caractérisé en ce qu'**un étranglement (14), contrôlé par un capteur de pression (15), est disposé après la dérivation dans la conduite de gaz support (19a).

26. Dispositif selon l'une quelconque ou plusieurs des revendications 17 à 25, **caractérisé en ce que** le dispositif comprenant le capteur (13) présente une chambre d'accumulation (43) utilement cylindrique, qui est délimitée par une paroi de fond (41) et une paroi de recouvrement, dans lequel le capteur (13) est positionné dans la paroi de recouvrement de manière à saillir dans la chambre et la paroi de fond (41) comprend une conduite d'amenée de gaz (42) dirigée sur le capteur (13) et conduisant uniquement dans la chambre et une conduite d'évacuation du gaz (41) dirigée également sur le capteur (13) et arrivant juste devant le capteur (13) et dans lequel la conduite d'amenée de gaz (42) est raccordée à la conduite de raccordement de gaz (13a) et la conduite d'évacuation du gaz (41) est raccordée à la conduite de raccordement de gaz (13b).

27. Dispositif selon l'une quelconque ou plusieurs des revendications 17 à 26, **caractérisé en ce que** l'on utilise une électrovanne à bascule 3/2 voies (11).

28. Dispositif selon la revendication 27, **caractérisé en ce que** la conduite de raccordement de gaz (13a) est raccordée à une conduite d'évacuation de gaz (60) de l'électrovanne à bascule (11) et les conduites de dérivation (11 a, 11b) sont raccordées chacune à une conduite d'amenée de gaz (58, 59) de l'électrovanne à bascule (11), dans lequel les conduites d'amenée de gaz (58, 59) peuvent être refermées tour à tour par une bascule à double bras (56).

29. Dispositif selon l'une quelconque ou plusieurs des revendications 17 à 28, **caractérisé en ce que** d'autres capteurs de gaz (3, 4, 5, 7) sont disposés dans la conduite de mélange de gaz de mesure (19).

30. Dispositif selon la revendication 29, **caractérisé en ce que** les autres capteurs de gaz sont disposés entre le régulateur de pression (2) et l'étranglement (6).
